# EUROPEAN PATENT SPECIFICATION

(11) **EP 3 419 649 B1**
(45) Date of publication and mention of the grant of the patent: **09.04.2025**
(21) Application number: 17716011.6
(22) Date of filing: 24.02.2017
(51) Int. Cl.: A61K 38/28, A61P 3/10, A61K 9/00, A61K 31/198

(54) **STABLE PHARMACEUTICAL COMPOSITIONS COMPRISING INSULIN GLARGINE AND INSULIN ASPART AND METHODS OF PREPARATION THEREOF**
STABILE PHARMAZEUTISCHE ZUSAMMENSETZUNGEN ENTHALTEND INSULIN GLARGIN UND INSULIN ASPART UND VERFAHREN ZU DEREN HERSTELLUNG
COMPOSITIONS PHARMACEUTIQUES STABLES COMPRENANT DE L'INSULINE GLARGINE ET DE L' INSULINE ASPART ET PROCÉDÉS DE PRÉPARATION DE CELLES-CI

(30) Priority: 25.02.2016 IN 201621006633; 25.02.2016 IN 201621006634
(43) Date of publication of application: 02.01.2019
(73) Proprietor: Wockhardt Limited, Mumbai 431006 (IN)
(72) Inventor: SAHIB, Maharaj Kishen, Mumbai Maharashtra 400078 (IN); BARHATE, Ganesh, Maharashtra, Pin - 414607 (IN); KATRGADDA, Mamatha, Krishna Andhra Pradesh 521156 (IN)
(74) Representative: Arnold & Siedsma
(86) International application number: PCT/IB2017/051069
(87) International publication number: WO 2017/145104

(56) References cited:
- EP-A1- 0 884 053
- WO-A1-2014/102623
- WO-A1-2015/044922
- AU-A1- 2012 247 063
- FR-A1- 2 978 918
- US-A1- 2008 039 365
- US-A1- 2013 178 415
- US-A1- 2015 265 683
- BAYNES B M ET AL: "Role of arginine in the stabilization of proteins against aggregation", BIOCHEMISTRY, AMERICAN CHEMICAL SOCIETY, US, vol. 44, no. 12, 1 March 2005 (2005-03-01), pages 4919 - 4925, XP002686707, ISSN: 0006-2960, [retrieved on 20050305], DOI: 10.1021/BI047528R

## Description

### TECHNICAL FIELD OF THE INVENTION

The invention is defined in claims 1 to 5 and relates to pharmaceutical composition comprising insulin glargine and amino acids. In particular, the present invention relates to a pharmaceutical composition comprising: (a) insulin glargine, (b) at least two amino acids and optionally (c) one or more pharmaceutically acceptable excipients.

### BACKGROUND OF THE INVENTION

Diabetes mellitus is a metabolic disorder in which the ability to utilize glucose is more or less completely lost. For decades, insulin has been used in the treatment of diabetes mellitus for which several insulin formulations have been developed. Currently available rapid acting insulin includes insulin lispro, insulin aspart and insulin glulisine. Fast acting insulin shows faster absorption and rapid onset of action i.e. within 30 min of administration. Thus, they are used for effective control of postprandial increase in the blood sugar level. These analogous are used for the treatment of type 1 (insulin-dependent) and type II (non-insulin-dependent) diabetes. For immediate and long term control of the glycemic level in the body, long acting insulin is given in combination with rapid acting insulin. Basal-bolus insulin therapy given as either multiple daily injections or by an insulin pump is a mainstay of diabetes treatment for achieving optimal glycemic control in type I diabetes. Attempts have been made to mix the different types of insulin in one injection in order to reduce the number of injections to be administered to a patient in the day. Due to difference in isoelectric points, mixing of different types of insulin prior to administration is not recommended as it leads to uncontrolled precipitation after mixing due to which it is difficult to predict the exact dose that has been administered by the patient.

Insulin glargine cannot be readily mixed with other insulin, insulin analogues or derivative having isoelectric points 4.0 to 5.7, because the mixture causes glargine to precipitate prior to injection and administration of precipitated insulin makes it virtually impossible to administer a known and reliable dose. Several warnings have been issued by the regulatory agencies against mixing long and rapid-acting insulin's together (insulin glargine, LANTUS; Sanofi-Aventis; available from http://www.lantus.com/hcp/closing.aspx; insulin detemir rDNA origin, brand name LEVEMIR drug insert; Novo Nordisk, Bagsvaerd, Denmark).

Kaplan W et al. (2004) discloses the effects of mixing glargine and short-acting insulin analogs on glucose control. Evans et al. (2011) discloses that neither insulin glargine nor insulin detemir are suitable for mixing with other insulin analogues as this mixing substantially alters their pharmacokinetic properties. Lucchesi Mb et.al. (2012) discloses mixing Insulin Lispro with Insulin glargine immediately before the subcutaneous injection decreases Insulin Lispro serum peak concentration without affecting the glycemic profile after 12 wk in this group with type I diabetes mellitus. PCT publication No. WO2016/001862 A1 discloses biphasic pharmaceutical composition comprising an insulin analogue, derivative or metabolite having isoelectric point between 5.8 to 8.5, zinc or salts thereof, isotonic agent optionally along with one or more pharmaceutically acceptable excipients. PCT publication No. WO2014/118355 A1 discloses stabilized pharmaceutical formulations of insulin analogues and/or insulin derivatives. PCT publication No. WO2015/044922 A1 discloses a pharmaceutical composition comprising readily dissociable molecular aggregates formed by combining an insulin, insulin analogue, derivative or metabolite having isoelectric point between 5.8 to 8.5 in combination with one or more an insulin, insulin analogue, derivative or metabolite having isoelectric point between 4.0 to 5.7 optionally along with one or more excipients. WO2014/102623A1 discloses a pharmaceutical composition comprising human insulin, analogues or derivatives thereof, at least one or more amino acids and a halogenide optionally along with one or more pharmaceutically acceptable excipient(s).

There is a need to develop a stable and compatible insulin glargine composition which can be readily mixed with another insulin, insulin analogue or derivative having isoelectric point between 4.0 and 5.7, and which will lead to reduction in the number of injections that a patient needs to administer.

### SUMMARY OF THE INVENTION

In a first aspect of the invention, there is provided a stable biphasic pharmaceutical composition comprising: (a) insulin glargine in a suspension form, (b) insulin aspart, (c) at least two amino acids in a weight ratio of about 1:2, and optionally, (d) one or more pharmaceutically acceptable excipients, wherein the pharmaceutical composition has a pH between about 6.0 to about 8.0. The two amino acids are arginine and isoleucine. In an embodiment, insulin glargine is present in about 40IU to about 500IU. In another embodiment, insulin aspart is present in about 40IU to about 500IU. In another embodiment, the one or more pharmaceutically acceptable excipients comprise buffer, solubilising agent, isotonic agent, preservative, antioxidant, pH modifying agent or a combination thereof. In another embodiment, the composition is stable for at least 6 months at 25°C and relative humidity of 60%.

In a second aspect of the invention, there is provided a method of preparing a stable biphasic pharmaceutical composition comprising: (a) insulin glargine in a suspension form, (b) insulin aspart, (c) arginine and isoleucine in a weight ratio of 1:2, and optionally (d) one or more pharmaceutically acceptable excipients, wherein the pharmaceutical composition has a pH between about 6.0 to about 8.0, wherein the said method comprises steps of:
(i) preparing a solution comprising insulin glargine having pH adjusted in between about 3 and about 4,
(ii) preparing a solution of sodium hydroxide (1N) and adding it to the solution of step (i) to obtain a suspension of insulin glargine having pH of about 7 to about 8,
(iii) preparing a solution of arginine and isoleucine and adding it into the suspension of step (ii) with mixing,
(iv) preparing a solution comprising insulin aspart having pH of about 6 to about 7, and
(v) mixing the suspension of step (iii) and solution of step (iv) to obtain the stable biphasic pharmaceutical composition.

### DETAILED DESCRIPTION OF THE INVENTION

The term "insulin" herein includes mammalian insulin, insulin human.

The term "insulin analogue" herein includes insulin NPH, insulin lispro, insulin lispro protamine, insulin glulisine, insulin aspart, insulin aspart protamine, Gly(A21) human insulin, Gly(A21) Lys(B28) human insulin, Gly(A21) Lys(B28) Pro(B29) human insulin, Gly(A21) Asp(B28).

The term "insulin derivative" herein includes B29-Nεmyristoyl-des(B30) human insulin (insulin detemir), Lys(B29)-Nε-(N-palmitoyl-.gamma.-glutamyl)-des(B30) human insulin (insulin degludec), B29-Nε-palmitoyl-des(B30) human insulin, B29-Nε-myristoyl human insulin, B29-Nε-palmitoyl human insulin, B28-Nε-myristoyl LysB28ProB29 human insulin, B28-Nε-palmitoyl LysB28ProB29 human insulin, B30-Nε-myristoyl-ThrB29LysB30 human insulin, B30-Nε-palmitoylThrB29LysB30 human insulin, B29-Nε-(N-lithocholyl-.gamma.-glutamyl)-des(B30) human insulin, B29-Nε- b(ω-carboxyheptadecanoyl)des(B30) human insulin, B29-Nε-( ω-carboxyheptadecanoyl) human insulin.

The term "stable" herein relates to a physical and/or chemical stability of pharmaceutical composition of insulin.

The term "compatible" herein relates to a physical and/or chemical compatibility of insulin glargine composition when mixed with another insulin, insulin analogue or derivative having isoelectric point between 4.0 and 5.7.

The term "biphasic" herein relates to a composition of insulin glargine and another insulin, insulin analogue or derivative having isoelectric point between 4.0 and 5.7, wherein insulin glargine is in suspension form and another insulin, insulin analogue or derivative having isoelectric point between 4.0 and 5.7 is in solution form.

The term "pharmaceutically acceptable excipients" herein relates to non-active pharmaceutical ingredients which are within the scope of sound medical judgment suitable for use in pharmaceutical products.

The term "isoelectric point" herein relates is the pH at which the insulin molecule carries no net electrical charge in the statistical mean.

For clarity, insulin glargine can also be denoted as Gly(A21), Arg(B31), Arg(B32) insulin human and insulin aspart can also be denoted as Asp(B28) insulin human.

In a first aspect of the invention, there is provided a stable biphasic pharmaceutical composition comprising: (a) insulin glargine in a suspension form, (b) insulin aspart, (c) arginine and isoleucine in a weight ratio of about 1:2, and optionally, (d) one or more pharmaceutically acceptable excipients, wherein the pharmaceutical composition has a pH between about 6.0 to about 8.0.

In an embodiment, the concentration of arginine or isoleucine ranges from about 1mM to 100mM, or about 2mM to 50mM, or about 3mM to 25mM. In an embodiment, the concentration of arginine is about 2mM to 20mM, or about 2mM to 10mM. Alternatively, the concentration of arginine is about 3mM, or about 4mM, or about 5mM, or about 6mM, or about 7mM, or about 8mM, or about 9mM. In an embodiment, the concentration of isoleucine is about 2mM to 20mM, or about 2mM to 15mM. Alternatively, the concentration of isoleucine is about 3mM, or about 4mM, or about 5mM, or about 6mM, or about 7mM, or about 8mM, or about 9mM, or about 10mM, or about 11mM, or about 12mM, or about 13mM, or about 14mM. Alternatively, the concentration of arginine is about 7mM and the concentration of isoleucine is about 14mM.

In still another embodiment, the pharmaceutical composition is suitable for parenteral administration such as intramuscular, subcutaneous, intradermal and intravenous administration.

In another embodiment, insulin glargine is present in about 40IU to about 500IU. Alternatively, insulin glargine is present in about 80IU to about 400IU, or about 100IU to about 300IU, or about 200IU to about 300IU. Alternatively, insulin glargine is present in about 50IU, or about 60IU, or about 70IU, or about 80IU, or about 90IU, or about 100IU, or about 110IU, or about 120IU, or about 130IU, or about 140IU, or about 150IU, or about 160IU, or about 170IU, or about 180IU, or about 190IU, or about 200IU, or about 250IU, or about 300IU, or about 350IU, or about 400IU, or about 450IU. Each of this concentration constitutes an alternate embodiment of the invention. In another embodiment, insulin aspart is present in about 40IU to about 500IU. Alternatively, insulin aspart is present in about 80IU to about 400IU, or about 100IU to about 300IU, or about 200IU to about 300IU. Alternatively, insulin aspart is present in about 50IU, or about 60IU, or about 70IU, or about 80IU, or about 90IU, or about 100IU, or about 110IU, or about 120IU, or about 130IU, or about 140IU, or about 150IU, or about 160IU, or about 170IU, or about 180IU, or about 190IU, or about 200IU, or about 250IU, or about 300IU, or about 350IU, or about 400IU, or about 450IU. Each of this concentration constitutes an alternate embodiment of the invention.

The compositions of insulin glargine and insulin aspart can be provided in the form of a stable and biphasic composition and in fixed dose proportion, as percentage, 90:10, or 85:15, or 80:20, or 75:25, or 70:30, 65:35, or 60:40, or 55: 45 or 50:50. In another embodiment, such fixed dose composition is stable for at least 6 months at 25°C and relative humidity of 60%.

In another embodiment, the stable and biphasic composition comprises insulin glargine in suspension form and wherein insulin glargine possess a particle size (D90) ranging from 2µm to 60µm, or about 5µm to 30µm, or about 10µm to 20µm.

In another embodiment, the one or more pharmaceutically acceptable excipients comprise buffer, solubilising agent, isotonic agent, preservative, antioxidant, pH modifying agent or a combination thereof.

The pH modifying agents as used herein refers to a combination of acid and alkali. The pH modifying agents can be selected from the group comprising of hydrochloric acid, o-phosphoric acid, citric acid, acetic acid, succinic acid, lactic acid, gluconic acid, tartaric acid, 1,2,3,4-butane tetracarboxylic acid, fumaric acid or malic acid. Alkali is selected from the group comprising of sodium hydroxide, potassium hydroxide, sodium hydroxide, ammonium hydroxide, magnesium oxide, calcium hydroxide, calcium carbonate, magnesium carbonate, magnesium aluminum silicates, diethanolamine, monoethanolamine, sodium carbonate, sodium bicarbonate or triethanolamine and combination thereof.

The solubilizing agent are selected from the group consisting of partial and fatty acid esters and ethers of polyhydric alcohols such as of glycerol, sorbitol, glycine and the like (Span^{®}, Tween^{®}, in particular Tween^{®} 20 and Tween^{®}80, Myrj^{®}, Brij^{®}, Cremophore^{®} or poloxamers, Pluronics^{®} and Tetronics^{®}), polysorbates (Tween^{™}), sodium dodecyl sulfate (sodium lauryl sulfate), lauryl dimethyl amine oxide, cetyltrimethylammonium bromide (CTAB), polyethoxylated alcohols polyoxyethylene sorbitan, Octoxynol (Triton X100^{™}), N,N-dimethyldodecylamine-N-oxide, hexadecyltrimethylammonium bromide (HTAB), polyoxyl 10 lauryl ether, Brij 721^{™}, bile salts (sodium deoxycholate, sodium cholate), polyoxyl castor oil (Cremophor^{™}), nonylphenol ethoxylate (Tergitol^{™}), cyclodextrins, lecithin and methylbenzethonium chloride (Hyamine^{™}). The concentration of solubilizing agent (preferred is glycine) is about 1mM to 200mM or about 70mM, or about 100mM.

The isotonic agent as used herein includes salts, e.g., sodium chloride, dextrose, lactose or combination thereof.

The preservative as used herein include, but are not limited to, benzoic acid, butylparaben, ethyl paraben, methyl paraben, propylparaben, sodium benzoate, sodium propionate, benzalkonium chloride, benzethonium chloride, benzyl alcohol, cetypyridinium chloride, chlorobutanol, phenol, phenylethyl alcohol, 2-penoxyethanol, phenyl mercuric nitrate, thimerosal, metacresol and combinations thereof. The concentration of preservative is about 0.1mg/mL to about 5mg/mL, or about 0.4mg/ml, or about 2.4mg/ml.

The antioxidant as used herein can be selected from the group comprising of ascorbate (sodium/acid), bisulite sodium, butylated hydroxy anisole (BHA), butylated hydroxy toluene (BHT), cysteine / cysteinate HCl, dithionite sodium, gentisic acid, gentisic acid ethanolamine, glutamate monosodium, glutathione, formaldehyde sulfoxylate sodium, metabisulite potassium, metabisulite sodium, methionine, monothioglycerol (thioglycerol), propyl gallate, sulfite sodium, tocopherol alpha, alpha tocopherol hydrogen succinate, thioglycolate sodium and combination thereof.

The buffer as used herein include, but are not limited to, phosphate, acetate, citrate or TRIS (i.e. 2-amino-2-hydroxymethyl-1,3-propanediol) buffer and corresponding salts.

In another embodiment of the invention, there is provided a stable biphasic pharmaceutical composition comprising: (a) insulin glargine in a suspension form, (b) insulin aspart, (c) arginine and isoleucine in a weight ratio of about 1:2, and optionally (d) one or more pharmaceutically acceptable excipients, wherein the pharmaceutical composition has a pH between about 6.0 to about 8.0, and wherein insulin glargine is present in about 40IU to about 500IU, and wherein inulin aspart is present in about 40IU to about 500IU, and wherein the one or more pharmaceutically acceptable excipients comprise buffer, solubilising agent, isotonic agent, preservative, antioxidant, pH modifying agent or combination thereof, and wherein the composition is stable for at least 6 months at 25°C and relative humidity of 60%.

In another embodiment of the invention, there is provided a stable biphasic pharmaceutical composition comprising: (a) insulin glargine in a suspension form, (b) insulin aspart, (c) arginine and isoleucine in a weight ratio of about 1:2, and optionally (d) one or more pharmaceutically acceptable excipients, wherein the pharmaceutical composition has a pH between about 6.0 to about 8.0, , and wherein insulin glargine is present in about 50IU, or about 60IU, or about 70IU, or about 80IU, or about 90IU, or about 100IU, or about 110IU, or about 120IU, or about 130IU, or about 140IU, or about 150IU, or about 160IU, or about 170IU, or about 180IU, or about 190IU, or about 200IU, or about 250IU, or about 300IU, or about 350IU, or about 400IU, or about 450IU, and wherein insulin aspart is present in about 50IU, or about 60IU, or about 70IU, or about 80IU, or about 90IU, or about 100IU, or about 110IU, or about 120IU, or about 130IU, or about 140IU, or about 150IU, or about 160IU, or about 170IU, or about 180IU, or about 190IU, or about 200IU, or about 250IU, or about 300IU, or about 350IU, or about 400IU, or about 450IU, and wherein the one or more pharmaceutically acceptable excipients comprise buffer, solubilising agent, isotonic agent, preservative, antioxidant, pH modifying agent or combination thereof, and wherein the composition is stable for at least 6 months at 25°C and relative humidity of 60%.

In another embodiment of the invention, there is provided a stable biphasic pharmaceutical composition comprising: (a) insulin glargine in a suspension form, (b) insulin aspart, (c) arginine and isoleucine in a weight ratio of about 1:2, and optionally (d) one or more pharmaceutically acceptable excipients, wherein the pharmaceutical composition has a pH between about 6.0 to about 8.0, and wherein insulin glargine and insulin aspart are present in a fixed dose proportion, as percentage, 90:10, or 85:15, or 80:20, or 75:25, or 70:30, 65:35, or 60:40, or 55: 45 or 50:50.

In another embodiment of the invention, there is provided a stable biphasic pharmaceutical composition comprising: (a) insulin glargine in a suspension form, (b) insulin aspart, (c) arginine and isoleucine in a weight ratio of about 1:2, and optionally (d) one or more pharmaceutically acceptable excipients, wherein the pharmaceutical composition has a pH between about 6.0 to about 8.0, and wherein insulin glargine and insulin aspart are present in a fixed dose proportion, as percentage, 90:10, or 85:15, or 80:20, or 75:25, or 70:30, 65:35, or 60:40, or 55: 45 or 50:50, and wherein the composition is stable for at least 6 months at 25°C and relative humidity of 60%.

In a second aspect of the invention, there is provided a method of preparing a stable biphasic pharmaceutical composition comprising: (a) insulin glargine in a suspension form, (b) insulin aspart, (c) arginine and isoleucine in a weight ratio of 1:2, and optionally (d) one or more pharmaceutically acceptable excipients, wherein the pharmaceutical composition has a pH between about 6.0 to about 8.0, wherein the said method comprises steps of:
(i) preparing a solution comprising insulin glargine having pH adjusted in between about 3 and about 4,
(ii) preparing a solution of sodium hydroxide (1N) and adding it to the solution of step (i) to obtain a suspension of insulin glargine having pH of about 7 to about 8,
(iii) preparing a solution of arginine and isoleucine and adding it into the suspension of step (ii) with mixing,
(iv) preparing a solution comprising insulin aspart having pH of about 6 to about 7, and
(v) mixing the suspension of step (iii) and solution of step (iv) to obtain the stable biphasic pharmaceutical composition.

### EXAMPLES

### EXAMPLE 1 (not according to the invention): Preparation of compatible insulin glargine (100IU/ml and 200IU/ml) solution composition

Example 1 elaborates composition and manufacturing process for composition of compatible insulin glargine having a pH of 3.5±0.2 that is ready to mix (before injection) with insulin aspart solution or insulin human solution having a pH of 7.2±0.2, in the proportion ranging from, as percentage, 90:10 to 50:50 (insulin glargine : insulin aspart or insulin human).

### Manufacturing process:

### (a) Compatible insulin glargine solution 100IU/ml and 200IU/ml, 500 ml:

| | | |
|---|---|---|
| Insulin glargine solution, 500 ml | | |
| Each ml contains: | | |
| Insulin Glargine | 3.6 mg (100IU) | 7.2mg (200IU) |
| 85% Glycerol | 10 mg | 10 mg |
| Zinc chloride | 30 µg | 60 µg |
| m-Cresol | 2.7 mg | 2.7 mg |
| Glycine | 100mM | 100mM |
| L-Arginine | 7.14mM | 7.14mM |
| L-Isoleucine | 14.28mM | 14.28mM |

Defined quantity of m-cresol and glycerol (85%) were dissolved in 10% final batch size of water for injection to prepare a preservative solution. The insulin glargine and zinc chloride were suspended in 5% final batch size of water for injection to obtain slurry. To prepare a solution of insulin glargine, hydrochloric acid (1N) was added in the insulin glargine slurry at 1 ml/g of insulin glargine concentration (v/w). The defined quantity of glycine was then added in the dissolved insulin glargine solution and followed by addition of the preservative solution. To this, while stirring, calculated quantities of L-arginine and L-isoleucine (in the weight ratio of 1:2) were added. The solution volume was made up to 80% of final formulation batch size with water for injection. The pH of solution was further adjusted to 3.5±0.2 using hydrochloric acid (1N) or sodium hydroxide (1N). The final volume was made up to 100% with water for injection. The resultant insulin glargine solution was sterile filtered using 0.2µ PES membrane filter and collected in a filling tank.

### (b) Insulin aspart solution 100IU/ml and 200IU/ml, 500 ml:

| | | |
|---|---|---|
| Insulin aspart solution, 500 ml | | |
| Each ml contains: | | |
| Insulin Aspart | 3.6 mg (100IU) | 7.2mg (200IU) |
| Phenol | 1.5 mg | 1.5 mg |
| 85% Glycerol | 20 mg | 20 mg |
| Zinc chloride | 30 µg | 60 µg |
| m-Cresol | 1.72 mg | 1.72 mg |
| di-Sodium hydrogen phosphate dihydrate | 1.25 mg | 1.25 mg |

In a separate container, a defined quantity of phenol, m-cresol, glycerol (85%) and di-Sodium hydrogen phosphate dihydrate were dissolved in 10% of final batch size volume of water for injection to form a preservative solution. The insulin aspart and zinc chloride were suspended in 5% final batch size of water for injection to obtain slurry. To prepare a solution of insulin aspart, hydrochloric acid (1N) was added in the insulin aspart slurry at 1 ml/g of insulin aspart concentration (v/w). The prepared preservative solution was further added to above insulin aspart solution. The volume of solution from above step was made up with sterile water for injection to 80% of final batch size. The solution pH was adjusted to 7.2±0.2 by using sodium hydroxide (1N) solution to prepare the insulin aspart solution. Post pH adjustment, volume of the above solution is made up with water for injection to 100% of final batch. Prepared insulin aspart solution was filtered using 0.2µ PES membrane filter and collected in another filling tank.

### (c) Insulin human solution 100IU/ml and 200IU/ml, 500ml:

| | | |
|---|---|---|
| Insulin human solution, 500 ml | | |
| Each ml contains: | | |
| Insulin human | 3.6 mg (100IU) | 7.2 mg (200IU) |
| Citric acid monohydrate | 0.0021 mg | 0.0021 mg |
| 85% Glycerol | 20 mg | 20 mg |
| Zinc chloride | 30 µg | 60 µg |
| m-Cresol | 2.5 mg | 2.5 mg |
| Tri-Sodium citrate | 0.19 mg | 0.19 mg |

In a separate container, defined quantity of m-cresol, glycerol (85%), citric acid monohydrate and tri-sodium citrate were dissolved in 10% of final batch size volume of water for injection to form a preservative solution. The insulin human and zinc chloride were suspended in 5% final batch size of water for injection to obtain slurry. To prepare a solution of insulin human, hydrochloric acid (1N) was added in the insulin human slurry at 1 ml/g of insulin human concentration (v/w). The prepared preservative solution was further added to above insulin human solution. The volume of solution from above step was made up with water for injection to 80% of final batch size. The solution pH was adjusted to 7.2±0.2 by using sodium hydroxide (1N) solution to prepare the insulin human solution. Post pH adjustment, volume of the above solution is made up with water for injection to 100% of final batch. Prepared insulin human solution was filtered using 0.2µ PES membrane filter and collected in another filling tank.

### (d) Preparation of a premix solution (before injection) containing insulin glargine and insulin aspart or insulin human (70/30):

The solution formulations of insulin glargine and insulin aspart or insulin human are ready to be mixed with each other in the proportion ranging from, as percentage, 90:10 to 50:50. Since these solutions are mixed before injection, therefore solutions are filled separately in the suitable container closure systems and can be mixed just before the injection in a suitable sterile container system. For instance, the final solution containing insulin glargine and insulin aspart or insulin human (100IU/ml, 70/30) was prepared by mixing 70ml of insulin glargine solution (100IU/ml) and 30ml of insulin aspart or insulin human solution (100IU/ml) and the final solution containing insulin glargine and insulin aspart or insulin human (200IU/ml, 70/30) was prepared by mixing 70 ml of insulin glargine solution (200IU/ml) and 30 ml of insulin aspart or insulin human solution (200IU/ml) in a suitable container has following composition:

| | | | | |
|---|---|---|---|---|
| Each ml contains: | 100IU/ml | 200IU/ml | 100IU/ml | 200IU/ml |
| Insulin Glargine | 70 IU | 140 IU | 70 IU | 140 IU |
| Insulin Aspart | 30IU | 60 IU | - | - |
| Insulin Human | - | - | 30IU | 60 IU |
| Phenol | 0.45 mg | 0.45 mg | - | - |
| 85% Glycerol | 13 mg | 13 mg | 13 mg | 13 mg |
| Zinc chloride | 30 µg | 60 µg | 30 µg | 60 µg |
| m-Cresol | 2.4 mg | 2.4 mg | 2.64 mg | 2.64 mg |
| di-Sodium hydrogen phosphate dihydrate | 0.375 mg | 0.375 mg | - | - |
| Tri-sodium citrate | - | - | 0.057 mg | 0.057 mg |
| Citric acid monohydrate | - | - | 0.0021 mg | 0.0021 mg |
| Glycine | 70mM | 70mM | 70mM | 70mM |
| L-Arginine | 5mM | 5mM | 5mM | 5mM |
| L-Isoleucine | 10mM | 10mM | 10mM | 10mM |
| pH | 3.5±0.2 | 3.5±0.2 | 3.5±0.2 | 3.5±0.2 |

### EXAMPLE 2 (not according to the invention): Comparative solution compositions of insulin glargine and insulin aspart (200IU/ml) and stability studies

Three comparative compositions were made: composition devoid of L-isoleucine (2A), and composition devoid of L-arginine (2B) and altering the weight ratio of L-arginine and L-isoleucine that is 1:1 (2C). These compositions were compared with the composition of Example 1 (solution composition of insulin glargine and insulin aspart, 200IU/ml, not according to the invention).

| Comparative compositions | | | | |
|---|---|---|---|---|
| Ingredients | Example 1 | Comparative composition A | Comparative composition B | Comparative composition C |
| Each ml contains: | | | | |
| Insulin Glargine | 140 IU | 140 IU | 140 IU | 140 IU |
| Insulin Aspart | 60 IU | 60 IU | 60 IU | 60 IU |
| Phenol | 0.45 mg | 0.45 mg | 0.45 mg | 0.45 mg |
| 85% Glycerol | 13 mg | 13 mg | 13 mg | 13 mg |
| Zinc chloride | 60 µg | 60 µg | 60 µg | 60 µg |
| m-Cresol | 2.4 mg | 2.4 mg | 2.4 mg | 2.4 mg |
| di-Sodium hydrogen | 0.375 mg | 0.375 mg | 0.375 mg | 0.375 mg |
| phosphate dihydrate | | | | |
| Glycine | 70mM | 70mM | 70mM | 70mM |
| L-Arginine | 5mM | 5mM | - | 5mM |
| L-Isoleucine | 10mM | - | 10mM | 5mM |
| pH | 3.5±0.2 | 3.5±0.2 | 3.5±0.2 | 3.5±0.2 |

In Example 1 compositions and comparative compositions, where the contact time (before injection in a syringe) for two components (insulin glargine and insulin aspart) is expected to be less than 10 seconds to about 5 minutes, the stability of acid labile insulin aspart part was carried up to 24 hours at 25°C and relative humidity of 60%. Before analysis, to separate the two insulin's components from each other, samples were passed through cation-exchange column. This was followed by analysis of related impurities using-"Method for insulin aspart related proteins and method for high molecular weight proteins- Indian Pharmacopoeia 2014"

| Stability of insulin aspart with insulin glargine in solution of example 1 (not according to the invention) and comparative compositions (pH 3.5±0.2, up to 24h, at 25°C and relative humidity of 60%) | | | | | | |
|---|---|---|---|---|---|---|
| Compositions | Time in Hours | High Molecular weight impurities | Related impurities | | | |
| | | | B28 isoAsp | A21 Asp and B3 Asp | B3 isoAsp | Others |
| Example 1 | Initial | 0.01 | 0.01 | 0.08 | 0.02 | 0.09 |
| | 2 | 0.01 | 0.01 | 0.11 | 0.03 | 0.14 |
| | 4 | 0.03 | 0.03 | 0.23 | 0.05 | 0.29 |
| | 8 | 0.04 | 0.08 | 0.41 | 0.1 | 0.53 |
| | 24 | 0.12 | 0.14 | 0.96 | 0.22 | 1.18 |
| Comparative Example 2A | Initial | 0.01 | 0.02 | 0.11 | 0.03 | 0.16 |
| | 2 | 0.06 | 0.04 | 0.38 | 0.05 | 0.34 |
| | 4 | 0.13 | 0.06 | 0.57 | 0.09 | 0.74 |
| | 8 | 0.32 | 0.09 | 0.86 | 0.19 | 1.34 |
| | 24 | 0.93 | 0.18 | 1.25 | 0.34 | 2.5 |
| Comparative Example 2B | Initial | 0.01 | 0.04 | 0.09 | 0.07 | 0.23 |
| | 2 | 0.04 | 0.07 | 0.14 | 0.11 | 0.4 |
| | 4 | 0.1 | 0.1 | 0.34 | 0.16 | 0.85 |
| | 8 | 0.24 | 0.16 | 0.77 | 0.31 | 1.51 |
| | 24 | 0.78 | 0.24 | 1.64 | 0.45 | 2.77 |
| Comparative Example 2C | Initial | 0.01 | 0.03 | 0.09 | 0.05 | 0.1 |
| | 2 | 0.03 | 0.05 | 0.17 | 0.09 | 0.19 |
| | 4 | 0.09 | 0.08 | 0.35 | 0.13 | 0.36 |
| | 8 | 0.2 | 0.13 | 0.62 | 0.19 | 0.62 |
| | 24 | 0.56 | 0.18 | 1.18 | 0.27 | 1.95 |

As evident from the stability data that, the formation of high molecular weight impurities and related impurities with respect to insulin aspart in a premix with insulin glargine are more in comparative composition 2A, 2B and 2C than in Example 1 composition (not according to the invention).

### EXAMPLE 3 (not according to the invention): Comparative solution compositions of insulin glargine and insulin human (200IU/ml) and stability studies

Three comparative compositions were made: composition devoid of L-isoleucine (3A), and composition devoid of L-arginine (3B) and altering the weight ratio of L-arginine and L-isoleucine that is 1:1 (3C). These compositions were compared with the composition of Example 1 (solution composition of insulin glargine and insulin human, 200IU/ml, not according to the invention).

| Comparative compositions | | | | |
|---|---|---|---|---|
| Ingredients | Example 1 | Comparative composition A | Comparative composition B | Comparative composition C |
| Each ml contains: | | | | |
| Insulin Glargine | 140 IU | 140 IU | 140 IU | 140 IU |
| Insulin Human | 60 IU | 60 IU | 60 IU | 60 IU |
| Glycine | 70 mM | 70 mM | 70 mM | 70 mM |
| 85% **Glycerol** | 13 mg | 13 mg | 13 mg | 13 mg |
| Zinc chloride | 60 µg | 60 µg | 60 µg | 60 µg |
| m-Cresol | 2.64 mg | 2.64 mg | 2.64 mg | 2.64 mg |
| Tri-sodium citrate | 0.057 mg | 0.057 mg | 0.057 mg | 0.057 mg |
| Citric acid monohydrate | 0.0021 mg | 0.0021 mg | 0.0021 mg | 0.0021 mg |
| L-Arginine | 5mM | 5mM | - | 5mM |
| L-Isoleucine | 10mM | - | 10mM | 5mM |
| pH | 3.5±0.2 | 3.5±0.2 | 3.5±0.2 | 3.5±0.2 |

In Example 1 compositions and comparative compositions, where the contact time (before injection in a syringe) for two components (insulin glargine and insulin human) is expected to be less than 10 seconds to about 5 minutes, the stability of acid labile insulin human part was carried up to 24 hours at 25°C and relative humidity of 60%. Before analysis, to separate the two insulin's components from each other, samples were passed through cation-exchange column. This was followed by analysis of related impurities using-"Method for insulin human related proteins and method for high molecular weight proteins- European Pharmacopoeia 8th edition, Pg 2491-2493".

| Stability of insulin human with insulin glargine in solution of example 1 and comparative compositions (pH 3.5±0.2, up to 24h, at 25°C and relative humidity of 60%) | | | | |
|---|---|---|---|---|
| Compositions | Time in Hours | High Molecular weight impurities | Related Impurities | |
| | | | A-21 desamido insulin | Others |
| Example 1 | Initial | 0.01 | 0.3 | 0.07 |
| | 2 | 0.01 | 0.11 | 0.13 |
| | 4 | 0.03 | 0.23 | 0.30 |
| | 8 | 0.04 | 0.14 | 0.68 |
| | 24 | 0.08 | 0.41 | 1.8 |
| Comparative Example 3A | Initial | 0.01 | 0.06 | 0.18 |
| | 2 | 0.04 | 0.18 | 0.37 |
| | 4 | 0.11 | 0.42 | 0.79 |
| | 8 | 0.25 | 0.64 | 1.42 |
| | 24 | 0.71 | 0.87 | 2.74 |
| Comparative Example 3B | Initial | 0.01 | 0.07 | 0.21 |
| | 2 | 0.05 | 0.12 | 0.42 |
| | 4 | 0.11 | 0.30 | 0.82 |
| | 8 | 0.29 | 0.62 | 1.59 |
| | 24 | 0.86 | 1.14 | 2.92 |
| Comparative Example 3C | Initial | 0.01 | 0.07 | 0.13 |
| | 2 | 0.05 | 0.14 | 0.44 |
| | 4 | 0.12 | 0.34 | 0.91 |
| | 8 | 0.29 | 0.57 | 1.72 |
| | 24 | 0.68 | 0.92 | 3.14 |

As evident from the stability data that, the formation of high molecular weight impurities and related impurities with respect to insulin human in a premix with insulin glargine are more in comparative composition 3A, 3B and 3C than in Example 1 composition of the invention.

### EXAMPLE 4: Biphasic composition of insulin glargine and insulin aspart (according to the invention) or insulin human (not according to the invention) (100IU/ml and 200IU/ml suspension)

Example 4 of elaborates composition and manufacturing process for composition of compatible insulin glargine suspension having a pH of 7.2±0.2 formulated with insulin aspart or insulin human solution having a pH 7.2±0.2. The insulin glargine and insulin aspart or insulin human can be mixed in the proportion ranging from, as percentage, 90:10 to 50:50 (insulin glargine : insulin aspart or insulin human).

### Manufacturing process:

### (a) Compatible insulin glargine suspension, 700 ml:

| | | |
|---|---|---|
| Insulin glargine suspension, 700 ml | | |
| Each ml contains: | | |
| Insulin Glargine | 3.6 mg (100IU) | 7.2 mg (200 IU) |
| 85% Glycerol | 10 mg | 10 mg |
| Zinc chloride | 30 µg | 60 µg |
| m-Cresol | 2.7 mg | 2.7 mg |
| Glycine | 100mM | 100mM |
| L-Arginine | 7.14mM | 7.14mM |
| L-Isoleucine | 14.28mM | 14.28mM |

(i) Defined quantity of m-cresol and glycerol (85%) were dissolved in 10% final batch size of water for injection to prepare a preservative solution. The insulin glargine and zinc chloride were suspended in 5% final batch size of water for injection to obtain slurry. To prepare a solution of insulin glargine, hydrochloric acid (1N) was added in the insulin glargine slurry at 1 ml/g of insulin glargine concentration (v/w). The defined quantity of glycine was then added in the dissolved insulin glargine solution and followed by addition of the preservative solution. The solution volume was made up to 30% of final formulation batch size with water for injection. The pH of solution was at this stage was 3.5±0.2. The resultant insulin glargine solution was sterile filtered using 0.2µ PES membrane filter and collected in sterile container X.
(ii) Defined quantity of sodium hydroxide (1N) solution was filtered using 0.2µ PES membrane filter and added slowly in a sterile container X, where insulin glargine solution was previously collected. This activity was performed to adjust the pH of insulin glargine solution to 7.2±0.2. This change in pH resulted in formation of insulin glargine suspension of controlled particle size. The volume of the suspension was made up to 60% final batch size.
(iii) Arginine : Isoleucine solution - In a separate container 8% final batch size volume of water for injection was taken. While stirring, calculated quantities of L-Arginine and L-Isoleucine (1:2 weight ratio) were added in the water for injection. The solution was mixed at 300 rpm till the complete dissolution of aforementioned amino acids. The solution pH was adjusted to 7.2±0.2 using hydrochloric acid (1N) or sodium hydroxide (1N). The prepared solution was filtered using 0.2µ PES membrane and added in a sterile container X with mixing. The volume of the parts present in sterile container X was made up to 70% final batch size.

### (b) Insulin aspart solution, 300 ml:

| | | |
|---|---|---|
| Insulin aspart solution, 300 ml | | |
| Each ml contains: | | |
| Insulin Aspart | 3.6 mg (100IU) | 7.2 mg (200IU) |
| Phenol | 1.5 mg | 1.5 mg |
| 85% Glycerol | 20 mg | 20 mg |
| Zinc chloride | 30 µg | 60 µg |
| m-Cresol | 1.72 mg | 1.72 mg |
| di-Sodium hydrogen phosphate dihydrate | 1.25 mg | 1.25 mg |

In a separate container, a defined quantity of phenol, m-cresol, glycerol (85%) and di-Sodium hydrogen phosphate dihydrate were dissolved in 10% of final batch size volume of water for injection to form a preservative solution. The insulin aspart and zinc chloride were suspended in 5% final batch size of water for injection to obtain slurry. To prepare a solution of insulin aspart, hydrochloric acid (1N) was added in the insulin aspart slurry at 1 ml/g of insulin aspart concentration (v/w). The prepared preservative solution was further added to above insulin aspart solution. The volume of solution from above step was made up with sterile water for injection to 25% of final batch size. The solution pH was adjusted to 7.2±0.2 by using sodium hydroxide (1N) solution to prepare the insulin aspart solution. Post pH adjustment, volume of the above solution is made up with water for injection to 30% of final batch.

### (c) Insulin human solution, 300 ml:

| | | |
|---|---|---|
| Insulin human solution, 300 ml | | |
| Each ml contains: | | |
| Insulin human | 3.6 mg (100 IU) | 7.2 mg (200IU) |
| Citric acid monohydrate | 0.0021 mg | 0.0021 mg |
| 85% Glycerol | 20 mg | 20 mg |
| Zinc chloride | 30 µg | 60 µg |
| m-Cresol | 2.5 mg | 2.5 mg |
| Tri-Sodium citrate | 0.19 mg | 0.19 mg |

In a separate container, defined quantity of m-cresol, glycerol (85%), citric acid monohydrate and tri-sodium citrate were dissolved in 10% of final batch size volume of water for injection to form a preservative solution. The insulin human and zinc chloride were suspended in 5% final batch size of water for injection to obtain slurry. To prepare a solution of insulin human, hydrochloric acid (1N) was added in the insulin human slurry at 1 ml/g of insulin human concentration (v/w). The prepared preservative solution was further added to above insulin human solution. The volume of solution from above step was made up with water for injection to 25% of final batch size. The solution pH was adjusted to 7.2±0.2 by using sodium hydroxide (1N) solution to prepare the insulin human solution. Post pH adjustment, volume of the above solution is made up with water for injection to 30% of final batch.

### (d) Preparation of a biphasic composition containing insulin glargine and insulin aspart or insulin human (70/30):

The suspension of insulin glargine and solution of insulin aspart or insulin human can be mixed with each other in the proportion ranging from, as percentage, 90:10 to 50:50. The final suspension containing insulin glargine and insulin aspart or insulin human (100IU/ml, 70/30) was prepared by mixing 700ml of insulin glargine suspension (100IU/ml) and 300ml of insulin aspart or insulin human solution (100IU/ml) and the final suspension containing insulin glargine and insulin aspart or insulin human (200IU/ml, 70/30) was prepared by mixing 700ml of insulin glargine suspension (200IU/ml) and 300ml of insulin aspart or insulin human solution (200IU/ml) in a suitable container and has following composition:

| | | | | |
|---|---|---|---|---|
| Each ml contains: | 100IU/ml | 200IU/ml | 100IU/ml | 2001U/ml |
| Insulin Glargine | 70 IU | 140 IU | 70 IU | 140 IU |
| Insulin Aspart | 30IU | 60 IU | - | - |
| Insulin Human | - | - | 30IU | 60 IU |
| Phenol | 0.45 mg | 0.45 mg | - | - |
| 85% Glycerol | 13 mg | 13 mg | 13 mg | 13 mg |
| Zinc chloride | 30 µg | 60 µg | 30 µg | 60 µg |
| m-Cresol | 2.4 mg | 2.4 mg | 2.64 mg | 2.64 mg |
| di-Sodium hydrogen phosphate dihydrate | 0.375 mg | 0.375 mg | - | - |
| Tri-sodium citrate | - | - | 0.057 mg | 0.057 mg |
| Citric acid monohydrate | - | - | 0.0021 mg | 0.0021 mg |
| Glycine | 70mM | 70mM | 70mM | 70mM |
| L-Arginine | 5mM | 5mM | 5mM | 5mM |
| L-Isoleucine | 10mM | 10mM | 10mM | 10mM |
| pH | 7.2 ±0.2 | 7.2 ±0.2 | 7.2 ±0.2 | 7.2 ±0.2 |

### EXAMPLE 5: Comparative biphasic compositions containing insulin glargine and insulin aspart (200IU/ml) and stability studies

Three comparative biphasic compositions were made: composition devoid of L-isoleucine (5A), and composition devoid of L-arginine (5B) and altering the weight ratio of L-arginine and L-isoleucine that is 1:1 (5C). These compositions were compared with invention composition of example 4 (Biphasic composition of insulin glargine and insulin aspart, 200IU/ml).

| Comparative compositions | | | | |
|---|---|---|---|---|
| Ingredients | Example 4 (according to the invention) | Comparative composition A | Comparative composition B | Comparative composition C |
| Each ml contains: | | | | |
| Insulin Glargine | 140 IU | 140 IU | 140 IU | 140 IU |
| Insulin Aspart | 60 IU | 60 IU | 60 IU | 60 IU |
| Phenol | 0.45 mg | 0.45 mg | 0.45 mg | 0.45 mg |
| 85% Glycerol | 13 mg | 13 mg | 13 mg | 13 mg |
| Zinc chloride | 60 µg | 60 µg | 60 µg | 60 µg |
| m-Cresol | 2.4 mg | 2.4 mg | 2.4 mg | 2.4 mg |
| di-Sodium hydrogen phosphate dihydrate | 0.375 mg | 0.375 mg | 0.375 mg | 0.375 mg |
| Glycine | 70mM | 70mM | 70mM | 70mM |
| L-Arginine | 5mM | 5mM | - | 5mM |
| L-Isoleucine | 10mM | - | 10mM | 5mM |
| pH | 7.2±0.2 | 7.2±0.2 | 7.2±0.2 | 7.2±0.2 |

In Example 4 (according to the invention) compositions and comparative compositions, where the contact time for two components (insulin glargine and insulin aspart) is expected to be at least 6 months, the stability of insulin glargine part was carried up to 6 months at 25°C and relative humidity of 60% (accelerated stability condition; considered as indicator of product stability at 2-8°C up to 24 months). Before analysis, to separate the two insulin's components from each other, samples were passed through cation-exchange column. This was followed by analysis of related impurities using-"Method for insulin glargine related proteins and method for high molecular weight proteins- United States Pharmacopoeia 38th edition, Pg 3876-3878".

| Stability of insulin glargine in biphasic composition of example 4 (according to the invention) with insulin aspart and comparative compositions (pH 7.2±0.2, up to 6 months, at 25°C and relative humidity of 60%) | | | |
|---|---|---|---|
| Compositions | Time in Month | High Molecular weight impurities | Related Impurities |
| Example 4 | Initial | 0.01 | 0.03 |
| | 1 | 0.05 | 0.05 |
| | 2 | 0.09 | 0.07 |
| | 4 | 0.13 | 0.11 |
| | 6 | 0.18 | 0.19 |
| Comparative Example 5A | Initial | 0.01 | 0.05 |
| | 1 | 0.12 | 0.15 |
| | 2 | 0.23 | 0.42 |
| | 4 | 0.35 | 0.89 |
| | 6 | 0.42 | 1.64 |
| Comparative Example 5B | Initial | 0.01 | 0.08 |
| | 1 | 0.14 | 0.21 |
| | 2 | 0.26 | 0.43 |
| | 4 | 0.4 | 0.95 |
| | 6 | 0.57 | 1.78 |
| Comparative Example 5C | Initial | 0.01 | 0.05 |
| | 1 | 0.1 | 0.11 |
| | 2 | 0.23 | 0.21 |
| | 4 | 0.4 | 0.46 |
| | 6 | 0.49 | 0.73 |

As evident from the stability data that, the formation of high molecular weight impurities and related impurities with respect to insulin glargine in a biphasic composition are more in comparative composition 5A, 5B and 5C than in Example 4 composition of the invention.

### EXAMPLE 6 (not according to the invention): Comparative biphasic compositions of insulin glargine and insulin human (200IU/ml) and stability studies

Three comparative biphasic compositions were made: composition devoid of L-isoleucine (6A), and composition devoid of L-arginine (6B) and altering the weight ratio of L-arginine and L-isoleucine that is 1:1 (6C). These compositions were compared with a composition of Example 4 (biphasic composition of insulin glargine and insulin human, 200IU/ml).

| Comparative compositions | | | | |
|---|---|---|---|---|
| Ingredients | Example 4 (not according to the invention) | Comparative composition A | Comparative composition B | Comparative composition C |
| Each ml contains: | | | | |
| Insulin Glargine | 140 IU | 140 IU | 140 IU | 140 IU |
| Insulin Human | 60 IU | 60 IU | 60 IU | 60 IU |
| Glycine | 70 mM | 70 mM | 70 mM | 70 mM |
| 85% Glycerol | 13 mg | 13 mg | 13 mg | 13 mg |
| Zinc chloride | 60 µg | 60 µg | 60 µg | 60 µg |
| m-Cresol | 2.64 mg | 2.64 mg | 2.64 mg | 2.64 mg |
| Tri-sodium citrate | 0.057 mg | 0.057 mg | 0.057 mg | 0.057 mg |
| Citric acid monohydrate | 0.0021 mg | 0.0021 mg | 0.0021 mg | 0.0021 mg |
| L-Arginine | 5mM | 5mM | - | 5mM |
| L-Isoleucine | 10mM | - | 10mM | 5mM |
| pH | 7.2±0.2 | 7.2±0.2 | 7.2±0.2 | 7.2±0.2 |

In Example 4 (not according to the invention) compositions and comparative compositions, where the contact time for two components (insulin glargine and insulin human) is expected to be at least 6 months, the stability of insulin glargine part was carried up to 6 months at 25°C and relative humidity of 60% (accelerated stability condition; considered as indicator of product stability at 2-8°C up to 24 months). Before analysis, to separate the two insulin's components from each other, samples were passed through cation-exchange column. This was followed by analysis of related impurities using-"Method for insulin glargine related proteins and method for high molecular weight proteins- United States Pharmacopoeia 38th edition, Pg 3876-3878".

| Stability of insulin glargine in biphasic composition of Example 4 (not according to the invention) with insulin human and comparative compositions (pH 7.2±0.2, up to 6 months, at 25°C and relative humidity of 60%) | | | |
|---|---|---|---|
| Compositions | Time in Month | High Molecular weight impurities | Related Impurities |
| Example 4 | Initial | 0.01 | 0.04 |
| | 1 | 0.04 | 0.06 |
| | 2 | 0.08 | 0.8 |
| | 4 | 0.11 | 0.13 |
| | 6 | 0.17 | 0.21 |
| Comparative Example 6A | Initial | 0.03 | 0.04 |
| | 1 | 0.14 | 0.13 |
| | 2 | 0.25 | 0.39 |
| | 4 | 0.34 | 0.85 |
| | 6 | 0.44 | 1.59 |
| Comparative Example 6B | Initial | 0.02 | 0.07 |
| | 1 | 0.11 | 0.23 |
| | 2 | 0.23 | 0.47 |
| | 4 | 0.42 | 0.93 |
| | 6 | 0.55 | 1.72 |
| Comparative Example 6C | Initial | 0.01 | 0.04 |
| | 1 | 0.12 | 0.10 |
| | 2 | 0.25 | 0.22 |
| | 4 | 0.43 | 0.45 |
| | 6 | 0.53 | 0.78 |

As evident from the stability data that, the formation of high molecular weight impurities and related impurities with respect to insulin glargine in a biphasic composition with insulin human are more in comparative composition 6A, 6B and 6C than in Example 4 (not according to the invention).

## Claims

1. A stable biphasic pharmaceutical composition comprising: (a) insulin glargine in a suspension form, (b) insulin aspart, (c) arginine and isoleucine in a weight ratio of 1:2, and optionally, (d) one or more pharmaceutically acceptable excipients, wherein the pharmaceutical composition has a pH between 6.0 to 8.0.

2. The pharmaceutical composition of claim 1, wherein insulin glargine is present in 40IU to 500IU.

3. The pharmaceutical composition of claim 1, wherein the one or more pharmaceutically acceptable excipients comprise buffer, solubilising agent, isotonic agent, preservative, antioxidant, pH modifying agent or a combination thereof.

4. The pharmaceutical composition of claim 1, wherein the composition is stable for at least 6 months at 25 "C and a relative humidity of 60%.

5. A method of preparing a stable biphasic pharmaceutical composition comprising: (a) insulin glargine in a suspension form, (b) insulin aspart, (c) arginine and isoleucine in a weight ratio of 1:2, and optionally (d) one or more pharmaceutically acceptable excipients, wherein the pharmaceutical composition has a pH between 6.0 to 8.0, wherein the said method comprises steps of:
(i) preparing a solution comprising insulin glargine having a pH adjusted in between 3 and 4,
(ii) preparing a solution of sodium hydroxide (1N) and adding it to the solution of step (i) to obtain a suspension of insulin glargine having a pH of 7 to 8,
(iii) preparing a solution of arginine and isoleucine and adding it into the suspension of step (ii) with mixing,
(iv) preparing a solution comprising insulin aspart having a pH of 6 to 7, and
(v) mixing the suspension of step (iii) and solution of step (iv) to obtain the stable biphasic pharmaceutical composition.

## Patentansprüche

1. Stabile zweiphasige pharmazeutische Zusammensetzung, umfassend: (a) Insulin glargin in einer Suspensionsform, (b) Insulin aspart, (c) Arginin und Isoleucin in einem Gewichtsverhältnis von 1 : 2 und optional (d) einen oder mehrere pharmazeutisch verträgliche Hilfsstoffe, wobei die pharmazeutische Zusammensetzung einen pH-Wert zwischen 6,0 und 8,0 aufweist.

2. Pharmazeutische Zusammensetzung nach Anspruch 1, wobei Insulin glargin in einer Menge von 40 IE bis 500 IE vorhanden ist.

3. Pharmazeutische Zusammensetzung nach Anspruch 1, wobei der eine oder die mehreren pharmazeutisch verträglichen Hilfsstoffe Puffer, Lösungsvermittler, isotonisches Mittel, Konservierungsmittel, Antioxidans, pH-regulierendes Mittel oder eine Kombination davon umfassen.

4. Pharmazeutische Zusammensetzung nach Anspruch 1, wobei die Zusammensetzung bei 25 °C und einer relativen Luftfeuchtigkeit von 60 % mindestens 6 Monate lang stabil ist.

5. Verfahren zum Herstellen einer stabilen zweiphasigen pharmazeutischen Zusammensetzung, umfassend: (a) Insulin glargin in einer Suspensionsform, (b) Insulin aspart, (c) Arginin und Isoleucin in einem Gewichtsverhältnis von 1 : 2 und optional (d) einen oder mehrere pharmazeutisch verträgliche Hilfsstoffe, wobei die pharmazeutische Zusammensetzung einen pH-Wert zwischen 6,0 und 8,0 aufweist, wobei das Verfahren die Schritte umfasst:
(i) Herstellen einer Lösung, umfassend Insulin glargin, das einen pH-Wert eingestellt zwischen 3 und 4 aufweist,
(ii) Herstellen einer Lösung aus Natriumhydroxid (1N) und Hinzufügen dieser zu der Lösung aus Schritt (i), um eine Suspension von Insulin glargin, die einen pH-Wert von 7 bis 8 aufweist, zu erhalten,
(iii) Herstellen einer Lösung aus Arginin und Isoleucin und Hinzufügen dieser in die Suspension aus Schritt (ii) unter Mischen,
(iv) Herstellen einer Lösung, umfassend Insulin aspart, das einen pH-Wert von 6 bis 7 aufweist, und
(v) Mischen der Suspension aus Schritt (iii) und der Lösung aus Schritt (iv), um die stabile zweiphasige pharmazeutische Zusammensetzung zu erhalten.

## Revendications

1. Composition pharmaceutique biphasique stable comprenant : (a) de l'insuline glargine sous forme de suspension, (b) de l'insuline asparte, (c) de l'arginine et de l'isoleucine dans un rapport pondéral de 1:2, et éventuellement, (d) un ou plusieurs excipients pharmaceutiquement acceptables, dans laquelle la composition pharmaceutique ayant un pH compris entre 6,0 et 8,0.

2. Composition pharmaceutique selon la revendication 1, dans laquelle l'insuline glargine est présente dans 40 UI à 500 UI.

3. Composition pharmaceutique selon la revendication 1, dans laquelle le un ou plusieurs excipients pharmaceutiquement acceptables comprennent un tampon, un agent solubilisant, un agent isotonique, un conservateur, un antioxydant, un agent modifiant le pH ou une combinaison de ceux-ci.

4. Composition pharmaceutique selon la revendication 1, dans laquelle la composition est stable pendant au moins 6 mois à 25 °C et à une humidité relative de 60 %.

5. Procédé de préparation d'une composition pharmaceutique biphasique stable comprenant : (a) de l'insuline glargine sous forme de suspension, (b) de l'insuline aspart, (c) de l'arginine et de l'isoleucine dans un rapport de poids de 1:2, et éventuellement, (d) un ou plusieurs excipients pharmaceutiquement acceptables, dans lequel la composition pharmaceutique a un pH compris entre 6,0 et 8,0, dans lequel ledit procédé comprend les étapes consistant à :
(i) préparer une solution comprenant l'insuline glargine dont le pH est ajusté entre 3 et 4,
(ii) préparer une solution d'hydroxyde de sodium (1N) et l'ajouter à la solution de l'étape (i) pour obtenir une suspension d'insuline glargine ayant un pH de 7 à 8,
(iii) préparer une solution d'arginine et d'isoleucine et l'ajouter à la suspension de l'étape (ii) en mélangeant,
(iv) préparer une solution comprenant de l'insuline asparte ayant un pH de 6 à 7, et
(v) mélanger la suspension de l'étape (iii) et la solution de l'étape (iv) pour obtenir la composition pharmaceutique biphasique stable.
